# EUROPEAN PATENT APPLICATION

(11) **EP 0 934 927 A1**
(43) Date of publication of application: **11.08.1999**
(21) Application number: 98200321.2
(22) Date of filing: 04.02.1998
(51) Int. Cl.: C07C 273/04

(54) **"Process and plant for the production of urea with high conversion yield and low energy consumption"**

(71) Applicant: UREA CASALE S.A., 6900 Lugano-Besso (CH)
(72) Inventor: Pagani, Giorgio, 6900 Lugano (CH)
(74) Representative: Zardi, Marco

(57) **Abstract**

In a process for the production of urea, substantially pure ammonia and carbon dioxide are reacted in a reaction space wherefrom there exits a reaction mixture which is sent to a urea recovery section. From the recovery section, a carbamate diluted solution is obtained which is submitted to stripping with recycling of vapours to the reaction space, on prior condensation of the same. This process allows to achieve a high conversion yield at low energy consumption and implementation costs.

## Description

### Application field

In its more general aspect, the present invention relates to a process for urea production.

More particularly, the invention relates to a process for urea production of the type comprising the following steps:
- feeding ammonia and carbon dioxide to a reaction space;
- reacting said ammonia with said carbon dioxide in said reaction space, obtaining thereby a reaction mixture comprising urea, carbamate and free ammonia in aqueous solution;
- feeding said mixture to a urea recovery section;
- separating in said recovery section said carbamate and said free ammonia in aqueous solution from urea.

The invention also relates to a plant for carrying out the aforesaid process as well as to a method for modernizing an existing urea plant, obtaining thereby a plant according to the invention.

In the description given below and in the appended claims, with the term: "modernizing", it is understood to mean the on-site modification of a pre-existing plant in order to improve its performance and to obtain, for instance, greater production capacity and/or greater conversion yield, as well as a reduction in energy consumption.

As is known with respect to urea production, the need is increasingly felt of having, on the one hand, plants of greater production capacity and operating flexibility, while requiring, on the other hand, lower investment and operating costs, in particular as concerns energy.

### Prior art

For this purpose, several processes have been proposed and applied in the field for the production of urea, mainly based on carrying out conversion reactions in a reaction space fed with ammonia (NH₃) and carbon dioxide (CO₂), and to which the unreacted substances contained in the urea solution leaving the reaction space, in particular ammonia, carbon dioxide, and diluted carbamate in aqueous solution, are recycled.

The plant for carrying out a process of this type comprises a urea recovery section downstream to a reaction space, to separate the unreacted substances to be recycled from the urea solution. Besides, the synthesis reaction in the reaction space is performed at high pressures, generally higher than 200 bar (220-240 bar), and with high NH₃/CO₂ mole ratios comprised between 3,6-4,0, with a conversion yield ranging from 64% and 70%.

In order to reduce the high energy consumption necessary for carrying out such processes, there has been subsequently proposed to perform the conversion reaction in two different reaction spaces having different yields and arranged in parallel to one another, as disclosed for instance in EP-A-0 479 103.

Also in this case, all the unreacted substances are recycled to one of the reaction spaces.

While, on the one hand, this recycling allows an almost complete recovery of valuable substances such as ammonia and carbon dioxide, on the other hand it involves the necessity of sending to the reactor high amounts of water (H₂O), which are extremely limiting for the global conversion yield of carbon dioxide into urea, which yield is generally comprised between 55% and 62%.

In urea production processes of an entirely different type, based on the technology of stripping with CO₂ or NH₃ the reaction mixture leaving the reaction space, and comprising a carbamate decomposition unit located between the reaction space and the urea recovery section, this problem has been recently solved by submitting to stripping the diluted carbamate solution leaving the urea recovery section, with recycling of vapours to the reaction space on prior condensation of the same, as disclosed in EP-A-0 796 244.

By way of comparison, the urea synthesis reaction is performed according to these processes at pressures generally comprised between 140-160 bar and with NH₃/CO₂ molar ratios lower than 3,2, which values are substantially lower than the working values of the processes to which the present invention refers.

### Summary of the invention

The technical problem underlying the present invention is accordingly to conceive and provide a process for urea production at high conversion yield, technically simple to carry out and requiring low energy consumption and operating costs.

The above result is achieved, according to the invention, by a process of the aforesaid type, characterised in that it further comprises the steps of:
- submitting at least a part of said carbamate and said free ammonia in aqueous solution obtained in said urea recovery section to a treatment of partial decomposition of carbamate and partial separation of free ammonia, obtaining a flow comprising ammonia and carbon dioxide in vapour phase, and a flow comprising residual carbamate in aqueous solution;
- submitting said flow comprising ammonia and carbon dioxide in vapour phase to an at least partial condensation, obtaining thereby a flow comprising concentrated carbamate in aqueous solution;
- feeding said concentrated carbamate to said reaction space.

In the description given below and in the appended claims, with the term: "concentrated carbamate in aqueous solution", it is understood to mean a carbamate solution having a very low water content (only a few percent points).

According to the invention, at least a part of the carbamate and free ammonia in aqueous solution leaving the urea recovery section is advantageously submitted to a partial decomposition treatment that allows to separate unreacted ammonia and carbon dioxide from a solution rich in water and comprising residual carbamate.

As a consequence, the unreacted substances to be recycled have a very low water content, and it is therefore possible to drastically limit the amount of water fed to the reaction space, allowing a high conversion yield.

Surprisingly, it has been found that the conversion yield achievable thanks to the process according to the invention, is not only higher than the conversion yield achievable by the process disclosed in EP-A-0 479 103, but is even higher than the typical conversion yields of processes operating with high NH₃/CO₂ molar ratios and at pressures higher than 200 bar, described hereinabove with reference to the prior art.

Obtaining a high conversion yield allows to markedly reduce the amount of carbamate and unreacted substances to be separated from the reaction mixture, allowing therefore a lightening of the sections located downstream of the reaction space, and obtaining therefore a high saving in investment, operating and maintenance costs of the plant designed to implement such process, as well as a reduction in energy and material consumption (for instance vapour)

In this respect, satisfactory results have been achieved by submitting at least 50% - for instance between 60% and 100% - of the carbamate in aqueous solution to the partial decomposition treatment.

According to a preferred and particularly advantageous embodiment of the present process, at least a part of the carbon dioxide fed to the reaction space is previously utilised as stripping agent during the step of partial decomposition of carbamate and partial separation of free ammonia.

In this way, it is possible to obtain a carbamate decomposition and a free ammonia separation that are very effective even at extremely high pressures, i.e. higher than 200 bar, that are generally utilised for this kind of processes.

In other words, this particular utilisation of carbon dioxide to be fed to the reaction space advantageously allows - independently on the working pressure that can be therefore even very high - to improve the step of decomposition of carbamate and separation of free ammonia coming from the urea recovery section, and facilitates consequently the separation of water from the unreacted substances recycled to the reaction space.

Preferably, at least 60% of the carbon dioxide fed to the reaction space is previously utilised as stripping agent during the step of partial decomposition of carbamate and partial separation of free ammonia.

Thanks to the present invention, the reaction between ammonia and carbon dioxide is advantageously carried out in the reaction space at a pressure comprised between 170 and 250 bar, preferably between 180 and 240 bar.

Besides, in order to achieve a high level of decomposition of carbamate and separation of free ammonia in aqueous solution, the decomposition and partial separation treatment is preferably carried out at a pressure substantially corresponding to the pressure of the reaction space.

Lastly, to improve and assist the condensation and separation steps of unreacted substances in the urea recovery section, the flow comprising residual carbamate in aqueous solution resulting from the treatment of partial decomposition of carbamate and partial separation of free ammonia is advantageously fed to such recovery section.

With reference to the present invention, it is worth stressing that being in condition of carrying out a partial decomposition of the carbamate and a separation of the free ammonia comprised in the aqueous solution of carbamate to be recycled to the reaction space, in a process of the type described above with reference to the prior art, with a reaction space working at pressures higher than 200 bar, was not evident at all faced to the teaching inferable from the prior art.

Actually, because of the very intrinsic characteristics of such a process, it was unthinkable - according to the prior art - to be in condition of effectively applying the technology disclosed in EP-A-0 796 244 with reference to CO₂ or NH₃ isobaric stripping processes, without causing thereby a worsening in conversion yield.

A reduction in working pressure in the reaction space to values of between 140 and 160 bar as was the case with the process disclosed in EP-A-0 796 244 would have allowed to obtain an effective decomposition of carbamate and separation of free ammonia coming from the urea recovery section, but would have at the same time caused - according to the constant teaching of the prior art - a corresponding drastic and unavoidable reduction in conversion yield.

On the other hand, keeping the working pressure in the reaction space unchanged - i.e. on the order of 200 bar - would have been substantially the same as giving up the advantages obtainable from the additional step of decomposing the recycled solution, since the decomposition of carbamate and the separation of free ammonia are very difficult, and in any case substantially reduced, at such pressures.

Only following the researches carried out by the applicant, the above technical problem could be solved by a process which provides for the separation of water from the flow of carbamate in aqueous solution leaving the urea recovery section and sent to the reaction space, in an extremely simple, economical and effective manner, without the aforesaid drawbacks of the prior art.

According to another aspect of the present invention, the technical problem set forth above is solved by a plant designed to implement the above mentioned urea production process comprising:
- a urea synthesis reactor;
- means for feeding ammonia and carbon dioxide to said urea synthesis reactor;
- a recovery section of a reaction mixture comprising urea, carbamate and free ammonia in aqueous solution leaving said urea synthesis reactor, in order to separate urea from the carbamate and free ammonia in aqueous solution;
   and characterised in that it comprises:
- a stripping unit for submitting at least a part of said carbamate and said free ammonia in aqueous solution obtained in said urea recovery section to a treatment of partial decomposition of carbamate and partial separation of free ammonia, obtaining thereby a flow comprising ammonia and carbon dioxide in vapour phase and a flow comprising residual carbamate in aqueous solution;
- means for condensing at least partially the vapours leaving said stripping unit, obtaining thereby a flow comprising concentrated carbamate in aqueous solution;
- means for feeding said concentrated carbamate to said urea synthesis reactor.

In accordance with the present invention the plants intended for carrying out the urea production process may be provided either new or by modifying pre-existing plants so as to obtain a production capacity expansion and at the same time improved performance from the energy consumption viewpoint.

According to another aspect, the present invention accordingly makes available a method for modernizing a urea production plant of the type comprising:
- a urea synthesis reactor;
- means for feeding ammonia and carbon dioxide to said urea synthesis reactor;
- a recovery section of a reaction mixture comprising urea, carbamate and free ammonia in aqueous solution leaving said urea synthesis reactor, in order to separate urea from the carbamate and free ammonia in aqueous solution;
   which method is characterised in that it comprises the steps of:
- providing a stripping unit for submitting at least a part of said carbamate and said free ammonia in aqueous solution obtained in said urea recovery section to a treatment of partial decomposition of carbamate and partial separation of free ammonia, obtaining thereby a flow comprising ammonia and carbon dioxide in vapour phase and a flow comprising residual carbamate in aqueous solution;
- providing means for condensing at least partially the vapours leaving said stripping unit, obtaining thereby a flow comprising concentrated carbamate in aqueous solution;
- providing means for feeding said concentrated carbamate to said urea synthesis reactor.

Further characteristics and advantages of the present invention are set forth in the detailed description of a preferred embodiment thereof given below by way of non-limiting example with reference to the annexed figure.

### Brief description of the figure

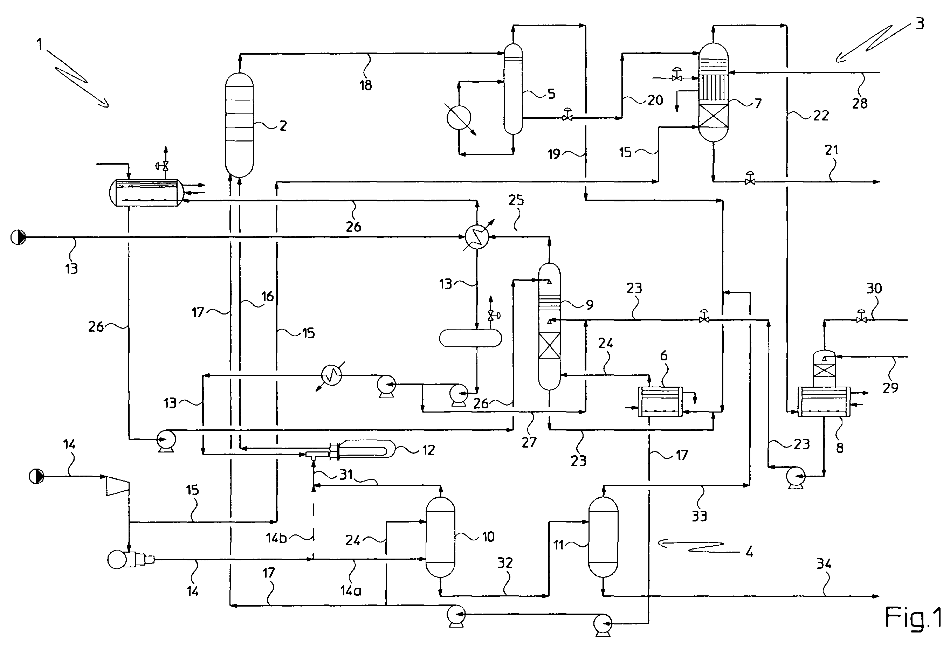

Figure 1 shows schematically and partially a urea production plant either realised anew or by modernizing a conventional plant for the implementation of the process according to the invention.

### Detailed description of a preferred embodiment

With the only aim of simplifying the description of the present invention, there is schematically represented in Figure 1 only a portion of a urea production plant, the omitted sections being not significant for the understanding of the present invention.

Besides, reference will be specifically made to the connection ducts of the different plant parts described in the following and shown in Figure 1, conventional per se, only wherever it is strictly necessary.

With reference to Figure 1, there is globally indicated by 1 a urea production plant according to the invention, of the total recycling type, i.e. wherein reagents are recycled to the reaction space.

Advantageously, plant 1 comprises a urea synthesis reactor 2 (or reaction space), a recovery section 3 of the urea produced, comprising in turn several apparatuses working at both high and low pressure - which will be described more in detail later on - and a carbamate decomposition and condensation section 4.

As will be seen later on, the urea production process according to the present invention allows to obtain in the urea synthesis reactor 2 a yield comprised between 70% and 78%.

An example of operating conditions of the urea synthesis reactor 2 obtainable with the present invention are: NH₃/CO₂ molar ratio 3,8, H₂O/CO₂ molar ratio 0,1, conversion yield 75%, pressure 220 bar, temperature 195°C.

Figure 1 does not show the vacuum section, which is an integrating part of the urea recovery section 3, and the sections of treatment and finishing of the concentrated urea solution, which are located downstream of section 3, said sections being of a known type and ininfluential for the purposes of the present invention

The urea recovery section 3 comprises a carbamate decomposer 5 and a condensation unit 6 at medium pressure (about 18 bar), a carbamate decomposer 7 and a condensation unit 8 at low pressure (about 4 bar), and an ammonia distillation column.

Section 4 of carbamate decomposition and condensation comprises in its turn a high pressure (170-250 bar) stripping unit or stripper 10 for the partial decomposition of carbamate and partial separation of free ammonia in aqueous solution coming from the urea recovery section 3, as well as a carbamate decomposer 11 (optional) and a carbamate condenser 12 for the decomposition of a flow comprising residual carbamate in aqueous solution coming from stripper 10 and for the absorption of vapours leaving the latter, respectively.

By 13 and 14, there are indicated the ducts that feed a flow comprising ammonia and a flow comprising carbon dioxide respectively.

From duct 14, a duct - indicated by 15 - branches off to feed a gas flow comprising carbon dioxide to the low pressure carbamate decomposer 7.

Carbon dioxide feeding reactor 2 is advantageously fed through ducts 14a and 14b to the stripping unit 10 and to the carbamate condenser 12, respectively. Duct 14b is entirely optional as - according to an embodiment of the invention - all the carbon dioxide to be sent to reactor 2 may be advantageously fed to stripper 10, and is therefore represented in Figure 1 by a broken line.

Reactor 2 is connected - at a bottom end and through ducts 16 and 17 - with the carbamate condenser 12, wherefrom a flow comprising concentrated carbamate in aqueous solution comes out, and with the condensation unit 6, wherefrom a flow comprising carbamate and free ammonia in aqueous solution comes out.

Reactor 2 is also connected - at the opposite top end and through duct 18 - with the high pressure carbamate decomposer 5, to which a reaction mixture comprising urea, carbamate and free ammonia in aqueous solution is fed.

The vapours comprising substantially water, ammonia and carbon dioxide obtained in the carbamate decomposer 5 are sent to the respective condensation unit 6 through duct 19, while the resulting urea solution is sent to the second carbamate decomposer 7 through duct 20.

In the carbamate decomposer 7, the urea solution is further concentrated and sent through duct 21 to the vacuum section, not shown. At the same time, the vapours obtained by this second decomposition are fed to the respective condensation unit 8 through duct 22.

The flow comprising carbamate and free ammonia in aqueous solution obtained in unit 8 is sent to the condensation unit 6 through duct 23, passing through the ammonia distillation column 9, wherefrom it is recycled, together with the flow comprising carbamate and free ammonia in aqueous solution obtained by condensation of the vapours coming from decomposer 5, through duct 17 to reactor 2 and advantageously through duct 24 to the carbamate decomposition and condensation section 4, and more precisely to stripper 10.

Particularly satisfactory results have been achieved by feeding at least 50%, preferably between 50-70%, of the flow comprising carbamate and free ammonia in aqueous solution leaving the condensation unit 6 to the carbamate decomposition and condensation section 4.

By the reference numerals 24-27 there are respectively indicated ducts for feeding or recycling flows comprising ammonia in vapour- respectively liquid phase to the ammonia distillation column 9.

By the reference numerals 28-30 there are respectively indicated ducts for feeding a vapour flow comprising H₂O, CO₂ and NH₃ coming from the treatment section (not shown) of the concentrated urea solution, an aqueous solution comprising CO₂ and NH₃ coming from the vacuum section (not shown), and a vapour flow comprising NH₃, CO₂ and H₂O to the vacuum section.

As such ducts 24-30 are conventional per se, and thus they will not be described with more details in the following of the description.

The flow comprising carbamate and free ammonia in aqueous solution advantageously fed through duct 24 to stripper 10 is submitted to a treatment of partial decomposition of carbamate and partial separation of free ammonia. In this way it is obtained a flow comprising ammonia and carbon dioxide in vapour phase, which is fed through duct 31 to the carbamate condenser 12, and a flow comprising residual carbamate in aqueous solution preferably recycled to the urea recovery section 3.

According to the example of Figure 1, a flow comprising residual carbamate in aqueous solution coming from stripper 10 is suitably fed through duct 32 to the carbamate decomposer 11, obtaining thereby a gas flow comprising CO₂ and NH₃ recycled through ducts 33 and 19 to the condensation unit 6, and a flow comprising very diluted carbamate in aqueous solution fed through duct 34 to the vacuum section, not shown.

In condenser 12, the flow comprising ammonia and carbon dioxide in vapour phase is advantageously condensed - at least partly - obtaining thereby a flow comprising concentrated carbamate in aqueous solution fed to reactor 2 through duct 16.

In this way it is possible to drastically reduce the amount of recycle water to be sent to the reaction space (reactor 2), allowing the latter to operate with extremely low H₂O/CO₂ molar ratios, to the full advantage of a high global yield of conversion of carbon dioxide into urea.

The high conversion yield obtained in reactor 2 also involves a reduction in the amount of carbamate produced and therefore - advantageously - a lightening of downstream sections, in particular the urea recovery section, which can therefore run in operating conditions much less heavy than design conditions, with ensuing high energy savings.

In other words, according to the process of the present invention, at least a part of the carbamate and free ammonia in aqueous solution obtained in the urea recovery section 3 is submitted (section 4, stripping unit 10) to a treatment of partial decomposition of carbamate and partial separation of free ammonia, obtaining thereby a flow comprising ammonia and carbon dioxide in vapour phase and a flow comprising residual carbamate in aqueous solution. The flow comprising ammonia and carbon dioxide in vapour phase is then submitted (section 4, condenser 12) to an at least partial condensation, obtaining thereby a flow comprising concentrated carbamate in aqueous solution, which is advantageously fed to the reaction space (reactor 2)

As stressed hereinabove, according to a preferred and particularly advantageous embodiment, shown in Figure 1, at least a part of the carbon dioxide fed to the reaction space (reactor 2) is previously utilised as stripping agent during the step of partial decomposition of carbamate and partial separation of free ammonia (section 4, stripping unit 10).

In this manner a very effective decomposition of the solution to be recycled to reactor 2 is obtained, independently on the operating pressure and therefore also at pressures higher than 200 bar.

Particularly satisfactory results in conversion yield and energy saving have been obtained by working in the inside of the reaction space, at a pressure preferably comprised between 180 and 140 bar.

Besides, the carbamate decomposition and condensation section 4 is advantageously caused to work at the same pressure as that of the reaction space.

In particular, at pressures comprised between 180 and 240 bar and by feeding carbon dioxide to the stripping unit 10, it is possible to obtain and effective carbamate decomposition and a subsequent condensation of the vapours comprising CO₂ and NH₃.

The plant of Figure 1 may be a new plant or - according to a preferred embodiment of the invention - it may be realised by modernizing a pre-existing urea production plant.

Advantageously, the modernizing of a urea production plant of the type comprising a urea synthesis reactor 2 and a recovering section 3 of a reaction mixture comprising urea, carbamate and free ammonia in aqueous solution coming from reactor 2 to separate urea from carbamate and the free ammonia in aqueous solution, is characterised by the steps of:
- providing a stripping unit 10 to submit al least a part of the carbamate and free ammonia in aqueous solution obtained in the urea recovery section 3 to a treatment of partial decomposition of carbamate and partial separation of free ammonia, obtaining thereby a flow comprising ammonia and carbon dioxide in vapour phase and a flow comprising residual carbamate in aqueous solution;
- providing means (carbamate condenser 12) for condensing at least partially the vapours leaving the stripping unit 10, obtaining thereby a flow comprising concentrated carbamate in aqueous solution; and
- providing means (duct 16) for feeding the concentrated carbamate to the urea synthesis reactor 2.

According to a particularly advantageous feature of the present invention, the modernizing method comprises the further step of providing means (14a) for feeding at least a part of the carbon dioxide to be fed to the synthesis reactor (2) to the stripping unit (10).

Preferably, such a method also comprises the step of:
- providing means (ducts 32-34) for feeding the flow comprising residual carbamate in aqueous solution from the stripping unit 10 to the urea recovery section 3.

In the description given below and in the appended claims, with the term: "means for feeding", it is understood to mean in general the various parts of a plant, such as for instance ducts, pumps and compressors, that serve to transport a liquid or gas fluid from one to another part of the plant.

Besides, with the term "means for condensing", it is understood to mean the apparatuses of the plant intended for the condensation (at least partial) of substantially gaseous flows, such as for instance the carbamate condenser 12.

## Claims

1. Process for the production of urea, of the type comprising the steps of:
- feeding ammonia and carbon dioxide to a reaction space;
- reacting said ammonia with said carbon dioxide in said reaction space, obtaining thereby a reaction mixture comprising urea, carbamate and free ammonia in aqueous solution;
- feeding said mixture to a urea recovery section;
- separating in said recovery section said carbamate and said free ammonia in aqueous solution from urea;
characterised in that it further comprises the steps of:
- submitting at least a part of said carbamate and said free ammonia in aqueous solution obtained in said urea recovery section to a treatment of partial decomposition of carbamate and partial separation of free ammonia, obtaining a flow comprising ammonia and carbon dioxide in vapour phase, and a flow comprising residual carbamate in aqueous solution;
- submitting said flow comprising ammonia and carbon dioxide in vapour phase to an at least partial condensation, obtaining thereby a flow comprising concentrated carbamate in aqueous solution;
- feeding said concentrated carbamate to said reaction space.

2. Process according to claim 1, characterised in that at least a part of said carbon dioxide fed to said reaction space is previously utilised as stripping agent during the step of partial decomposition of carbamate and partial separation of free ammonia.

3. Process according to claims 1 and 2, characterised in that said reaction between ammonia and carbon dioxide is performed in said reaction space at a pressure comprised between 170 and 250 bar, preferably between 180 and 240 bar.

4. Process according to claims 1 and 2, characterised in that the treatment of partial decomposition of carbamate and partial separation of free ammonia is performed at a pressure substantially corresponding to the pressure of said reaction space.

5. Process according to claim 1, characterised in that it further comprises the step of:
- feeding said flow comprising residual carbamate in aqueous solution resulting from the treatment of partial decomposition of carbamate and partial separation of free ammonia to said urea recovery section.

6. Process according to claim 1, characterised in that at least 50% of said carbamate in aqueous solution is submitted to the treatment of partial decomposition.

7. Process according to claim 2, characterised in that at least 60% of said carbon dioxide fed to said reaction space is previously utilised as stripping agent during the step of partial decomposition of carbamate and partial separation of free ammonia.

8. Plant for urea production comprising:
- a urea synthesis reactor (2);
- means (13, 14) for feeding ammonia and carbon dioxide to said urea synthesis reactor (2);
- a recovery section (3) of a reaction mixture comprising urea, carbamate and free ammonia in aqueous solution leaving said urea synthesis reactor (2), in order to separate urea from the carbamate and free ammonia in aqueous solution;
characterised in that it comprises:
- a stripping unit (10) for submitting at least a part of said carbamate and said free ammonia in aqueous solution obtained in said urea recovery section (3) to a treatment of partial decomposition of carbamate and partial separation of free ammonia, obtaining thereby a flow comprising ammonia and carbon dioxide in vapour phase and a flow comprising residual carbamate in aqueous solution;
- means (12) for condensing at least partially the vapours leaving said stripping unit, obtaining thereby a flow comprising concentrated carbamate in aqueous solution;
- means (16) for feeding said concentrated carbamate to said urea synthesis reactor.

9. Plant according to claim 8, characterised in that it further comprises:
- means (14a) for feeding at least a part of said carbon dioxide to be fed to the synthesis reactor (2) to said stripping unit (10).

10. Plant according to claim 8, characterised in that it further comprises:
- means (32-34) for feeding said flow comprising residual carbamate in aqueous solution from said stripping unit (1) to said urea recovery section (3)

11. Method for modernizing a plant for urea production of the type comprising:
- a urea synthesis reactor (2);
- means (13, 14) for feeding ammonia and carbon dioxide to said urea synthesis reactor (2);
- a recovery section (3) of a reaction mixture comprising urea, carbamate and free ammonia in aqueous solution leaving said urea synthesis reactor (2), in order to separate urea from the carbamate and free ammonia in aqueous solution;
characterised in that it comprises:
- providing a stripping unit (10) for submitting at least a part of said carbamate and said free ammonia in aqueous solution obtained in said urea recovery section (3) to a treatment of partial decomposition of carbamate and partial separation of free ammonia, obtaining thereby a flow comprising ammonia and carbon dioxide in vapour phase and a flow comprising residual carbamate in aqueous solution;
- providing means (12) for condensing at least partially the vapours leaving said stripping unit, obtaining thereby a flow comprising concentrated carbamate in aqueous solution;
- providing means (16) for feeding said concentrated carbamate to said urea synthesis reactor.

12. Method according to claim 11, characterised in that it further comprises the step of:
- providing means (14a) for feeding at least a part of said carbon dioxide to be fed to the synthesis reactor (2) to said stripping unit (10).

13. The method according to claim 11, characterised in that it further comprises the step of:
- providing means (32-34) for feeding said flow comprising residual carbamate in aqueous solution from said stripping unit (1) to said urea recovery section (3)
